# EUROPEAN PATENT SPECIFICATION

(11) **EP 2 772 215 B1**
(45) Date of publication and mention of the grant of the patent: **21.10.2015**
(21) Application number: 13181884.1
(22) Date of filing: 27.08.2013
(51) Int. Cl.: A61B 17/68, A61B 17/84, A61B 17/72

(54) **Orthopedic implant for treatment of bone deformities**
Orthopädisches Implantat zur Behandlung von Knochendeformationen
Implant orthopédique pour le traitement de malformations osseuses

(30) Priority: 27.02.2013 US 201361769774 P; 28.02.2013 US 201313780126
(43) Date of publication of application: 03.09.2014
(73) Proprietor: Bonfix Ltd., 91391 Jerusalem (IL)
(72) Inventor: Robinson, Dror, 99788 M.P. Shemson (IL); Shahar, Mark, Tel-Aviv 6939209 (IL); Barkai, Nir, 44221 Kfar Saba (IL); Schon, Lew, Pikesville, MD Maryland 21208-3312 (US)
(74) Representative: Pearl Cohen Zedek Latzer Baratz UK LLP

(56) References cited:
- WO-A1-2010/093696
- WO-A1-2012/029008
- US-A1- 2009 182 336

## Description

### FIELD OF THE INVENTION

The present invention relates generally to devices for orthopedic surgical procedures, and specifically to devices implantable into the bones.

### BACKGROUND OF THE INVENTION

"Hallux valgus" or "hallux abducto valgus" is a common disorder of the forefoot, which is associated with bunion deformity. The term refers to an abnormal slant of the big toe relative to the second toe. It is one of the most common pathologic conditions affecting the foot and toes.

Hallux valgus results from a medial deviation of the first metatarsal bone and lateral deviation and/or rotation of the big toe (hallux), with or without medial soft-tissue enlargement of the first metatarsal head (bunion). In normal feet, the angle between the first and second metatarsal bones (known as the Inter-Metatarsal Angle, or IMA) is typically in the range of 6-9°. In hallux valgus, this angle may increase to more than 12° in moderate cases and more than 16° in severe cases. Treatment of hallux valgus often includes surgical intervention to reduce the inter-metatarsal angle.

A number of surgical devices and techniques have been developed for reduction of the inter-metatarsal angle. For example, U.S. Patent Application Publication 2010/0152752, discloses a method and apparatus for bunion repair using a suture-passing K-wire. (A K-wire, or Kirschner wire, is a sharpened, smooth metal pin, widely used in orthopedic surgery, which is driven into the bone using a drill.) The K-wire is used to pass a suture through the first and second metatarsal bones. The first and second metatarsals are pushed together to correct the inter-metatarsal angle deformity, and the suture is tied in this position to hold anchor buttons against the bones. Arthrex, Inc. (Naples, Florida) offers a commercial product of this sort, known as the Mini TightRope, which is also described in U.S. Patent 7,875,058.

PCT International Publication WO 2009/018527 describes a fixation and alignment device for use in orthopedic surgery, for the correction of bone deformities. The device is part of an anchoring system that is said to be suitable for surgical repair of hallux valgus and other conditions. The system is used to anchor two or more sections of bone or other body parts and to align one section relative to another.

PCT International Publication WO 2010/093696 describes devices for treating hallux valgus using dynamic tensioning components or heat shrinkable components to urge two metatarsals together to treat a bone deformity. The dynamic tensioning component exhibits elasticity and has a tensioned state and an untensioned state. In the tensioned state, the component urges first and second anchors, attached to the first and second metatarsal bones, toward each other.

WO/2012/029008 discloses an implantable device including a first anchor, configured to be implanted inside a first bone and a second anchor, configured to be implanted inside a second bone, adjacent to the first bone. A cord connects the first and second anchors. A shock absorber contained within at least one of the first and second anchors is coupled to the cord so as to deform in response to a force exerted on the cord. The anchor is flute shape to prevent bone to cord contact. The shock absorber may also include a force adjuster. The device may include an adjustment mechanism, which is operable, after implantation of the first and second anchors in the first and second bones, to adjust a length of the cord extending between the first and second anchors.

### SUMMARY OF THE INVENTION

Embodiments of the present invention that are described herein below provide a surgical device implantable into the bones.

There is therefore provided, in accordance with an embodiment of the present invention, an implantable device, comprising (a) a proximal anchor, configured to be implanted inside a first metatarsal (the first bone); said anchor having a tail collar which is larger than an opening in said metatarsal in which said proximal anchor is implanted; (b) a distal anchor, configured to be implanted inside (the second bone) e.g. a second metatarsal, adjacent to said first metatarsal; said distal anchor having a male thread; (c) a nut threadable onto said male thread of the distal anchor is possible; and (d) a cord mechanically interconnecting said proximal and distal anchors. At least one of said anchors is provided with a shock absorber.

It is a core purpose of the present invention is to provide said shock absorber comprising an elongate housing positioned within said anchor. The shock absorber accommodates a cord holder mechanically connected to said housing via a damping member, thereat said cord holder is can be freely rotatable within said housing to prevent said cord from twist kinking when said housing is rotated to adjust a tension force.

It is another object of the present invention to provide the damping member which is a spring.

It is another object of the present invention to provide a cable with changing amounts of tensions that acts as a ligament reconstruction support allowing guided tissue regeneration of inter-osseous ligament for example but not limited to the inter-metatarsal ligament. This cable may be coated with tissue growth enhancing material to simulate ligament reconstruction.

It is a further object of the present invention to provide the spring configured for operation under compression or extension.

It is a further object of the present invention to provide the at least one anchor provided with at least one tab deployable into a cortical area of a bone after implanting said anchor into.

In accordance with the present invention, a cord tension force is adjustable due to thread displacement of said shock absorber along said anchor.

It is a further object of the present invention to provide the said proximal anchor having a collar.

It is a further object of the present invention to provide the distal anchor having a male thread configured for threading a fixating nut at said thread to the rearward of said second metatarsal. However the distal anchor might also lack such a thread or have a female thread,

It is a further object of the present invention to provide the distal anchor having a female thread configured for threading an extension rod for guiding said nut to be threaded onto said distal anchor.

It is a further object of the present invention to provide the shock absorber comprising a telescopic element. It is a further object of the present invention to provide the shock absorber comprising a deformable sleeve that acts as a spring.

It is a further object of the present invention to provide the shock absorber comprising a deformable polymer bottleneck member.

In another embodiment of the present invention, the proximal or distal anchors or both might be inserted either oblique to or in parallel with the longitudinal axis of the bone or even perpendicular to it.

It is a further object of the present invention to provide a set for treating *Hallux Abducto Vagus*. The aforesaid set comprises(a) an implantable device further comprising: (i) a proximal anchor, configured to be implanted inside a first metatarsal; said anchor having a tail collar which is larger than an opening in said metatarsal in which said proximal anchor is implanted; (ii) a distal anchor, configured to be implanted inside a second metatarsal, adjacent to said first metatarsal; said distal anchor having a male thread; (iii) a nut threadable onto said male thread of the distal anchor; (iv) a cord mechanically interconnecting said proximal and distal anchors; at least one of said anchors provided with a shock absorber; (b) a K-wire drilling guide; and (c) a drilling device.

It is a further object of the present invention to provide the set comprising pliers configured for deploying said tab into said cortical area of a bone.

It is a further object of the present invention to provide the implantable device configured for an intramedullary insertion, without a male thread and nut threadable onto said male thread. It is also described herein a method of surgically treating *Hallux Abducto Vagus* (HAV).The aforesaid method comprises the steps of: (a) providing a set treating HAV further comprising (i) an implantable device, comprising:(1) a proximal anchor, configured to be implanted inside a first metatarsal; said anchor having a tail collar which is larger than an opening in said metatarsal in which said proximal anchor is implanted; (2) a distal anchor, configured to be implanted inside a second metatarsal, adjacent to said first metatarsal; said distal anchor having a male thread; (3) a nut threadable onto said male thread of the distal anchor; (4) a cord mechanically interconnecting said proximal and distal anchors; at least one of said anchors provided with a shock absorber; (ii) a K-wire drilling guide; (iii) a drilling device; said shock absorber comprises an elongate housing threadly positioned within said anchor; said shock absorber accommodates a cord holder mechanically connected to said housing via a damping member, thereat said cord holder is freely rotatable within said housing to prevent said cord from twist kinking when said housing is rotated to adjust a tension force; (b) positioning said a K-wire drilling guide at the patient's foot such that said drilling device is directed for drilling first and second metatarsals; (c) making openings in said first and second metatarsals; (d) successively inserting said implantable device into obtained holes in said first and second metatarsals such that said proximal anchor is mechanically fixated in an opening in said first metatarsal while said distal anchor is shoved through an opening in said second metatarsal; (e) threading said nut at said male thread shoved through an opening in said second metatarsal to the rearward of said second metatarsal.

Also described herein is a method comprising a step of damping mechanical effects during patient's ambulation by means of said shock absorber comprising an elongate housing threadly positioned within said anchor. The method comprises a step of adjusting a cord tension force by means of rotating said housing of said shock absorber such that said cord holder is freely rotatable within said housing to prevent said cord from twist kinking at said step of adjusting the cord tension force.

The present invention will be more fully understood from the following detailed description of the embodiments thereof, taken together with the drawings in which:

The present invention could be used for joining any two bones of the body including for example the tibia and fibula, the clavicle and the acromion, the clavicle and the coracoid process, the radius to ulna, or any of the carpal and tarsal bones.

The components could be implanted in an intramedullary location parallel or transverse or oblique to the bone.

A unique property of the variable tensioned cable is its ability to act as a tissue regenerating component supporting guided tissue regeneration of ligaments around the cable due to the forces on the cable as well as in some embodiments the specialized surface properties or layer enveloping the cable.

### BRIEF DESCRIPTION OF THE DRAWINGS

Fig. 1 is a schematic top view of the bones of a foot, in which a device for reducing inter-metatarsal angle has been implanted in accordance with an embodiment of the present invention;
Fig. 2 is a schematic cross-sectional view of an implantable device;
Fig. 3 is a schematic view of the device provided with an extension shaft;
Fig. 4 is a schematic view of the device provided with a nut and a locking tool;
Fig. 5 is a schematic view of the device provided with a handle;
Fig. 6 is a schematic view of the device provided with an adjusting tool fitted into the device;
Fig. 7 is a schematic view of the device provided with locking pliers;
Fig. 8 is a phantom view of a housing of a proximal anchor;
Fig. 9 is a scheme illustrating a preparatory stage in a procedure for device implantation;
Figs. 10-13 are schematic, pictorial views of the first and second metatarsal bones showing successive stages of the surgical procedure.

### DETAILED DESCRIPTION OF EMBODIMENTS

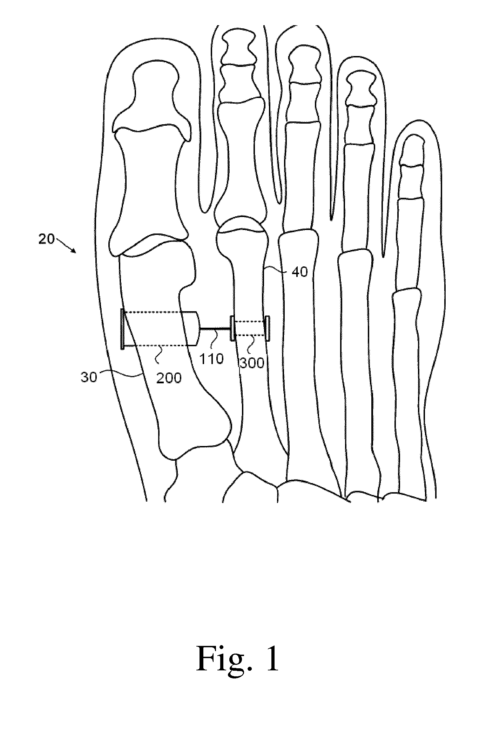

Reference is now made to Fig. 1 presenting a schematic top view of the bones of a foot, in which a device 20 for reducing the inter-metatarsal angle has been implanted in accordance with an embodiment of the present invention. Device 20 comprises a first anchor 200, which is implanted in a first metatarsal bone 30, and a second anchor 300, implanted in a second metatarsal bone 40. The anchors are connected by a flexible or rigid cord 110, which extends between the anchors (and thus between the two metatarsal bones 30 and 40). An adjustment mechanism, which is described in detail below and herein, enables the surgeon, after implantation of the anchors in the bones, to adjust the length of the cord extending between the first and second anchors. The surgeon can thus modify the inter-metatarsal distance, i.e. the IMA, meaning the angle between the respective axes of bones 30 and 40.

In the description that follows, it is assumed, for clarity of explanation, that the "proximal anchor" is implanted in the first metatarsal, while the "distal anchor" is implanted in the second metatarsal. In alternative embodiments, however, certain of the functions and features of the two anchors may be exchanged. Therefore, in the present patent application and in the claims, it should be understood that the terms "proximal" and "distal" are used arbitrarily in relation to the anchors and do not signify which anchor is to be implanted in which of the bones unless the specific context in which the terms are used indicates otherwise.

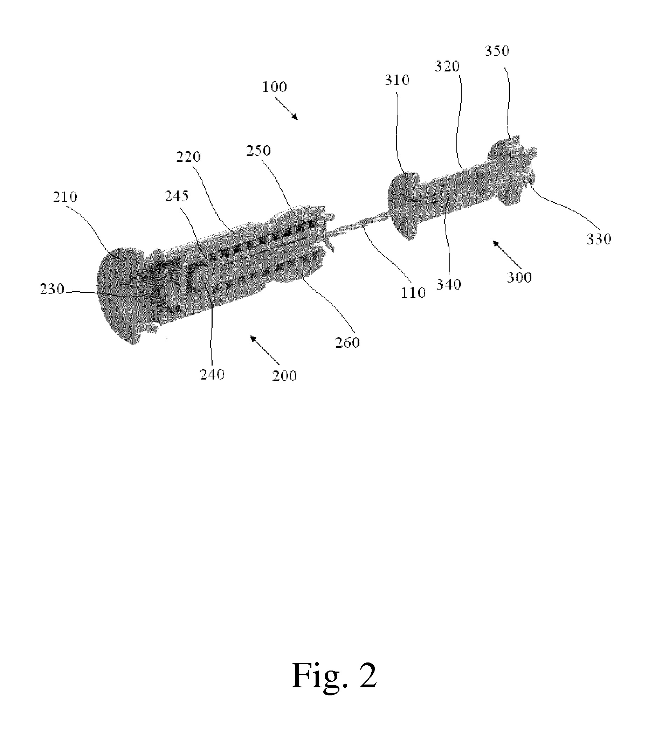

Reference is now made to Fig. 2 showing a cross-sectional view of an implantable device 100. The aforesaid device comprises a proximal anchor 200configured to be implanted inside a first metatarsal and a distal anchor 300configured to be implanted inside a second metatarsal. The proximal and distal anchors are interconnected by a cord 110. The proximal anchor 200 has (i) a collar 210 which is larger than an opening in the first metatarsal in which said proximal anchor is implanted; and (ii) a cylindrical portion 220. A shock absorber comprises an elongate housing 230threadly positioned within the anchor 200.A cord holder 245 is freely rotatable within said housing 230 to prevent said cord from twist kinking when said housing is rotated to adjust a tension force. The cord holder 245 have a passage there within with a bottleneck fastening a body 240 mechanically connected to the cord 110. A damping element (spring 250) is kinematically between the cord holder 245 and the housing 230 rigidly connected with the proximal anchor 200. A nut 260 is screwed onto the housing 230 and holds the spring 250 within the housing 230.

Similar to described, a body 340 connected to the cord 110 is fastened in the distal anchor 300. The aforesaid anchor 300 has a collar 310, a cylindrical portion 320 provided with a male thread 330. A nut 350 is designed for fixating the anchor 300 within the opening in the second metatarsal bone 40 (not shown).

It should be emphasized that, according to the present invention, the implant 100 consists of two anchors. The distal anchor 300 is of minor diameter in comparison with the proximal anchor 200. Openings in the first and second metatarsal bones 30 and40 are made such that the distal anchor 300 freely passes through the first metatarsal and settles into the opening in the second metatarsal tightly. Respectively, the proximal anchor 200 into the opening in the first metatarsal tightly.

Anchors 200 and 300 are made from a rigid biocompatible material, such as 316LVM-type stainless steel or titanium alloy, with a cylindrical shape for insertion into cylindrical bores that are drilled in the bones. For example, anchor 200 may be 12 mm long and 6 mm in diameter, while anchor 300 is 11 mm long and 3 mm in diameter. Alternatively, other dimensions may be chosen depending, inter alia, on the dimensions and condition of the bones in which the anchors are to be implanted. Anchors 200 and 300 may be coated with a bone growth promoter, such as hydroxyapatite.

Cord 110 may comprise any flexible (though inelastic), biocompatible material of sufficient strength to withstand the forces exerted by and on the bones of the foot. The cord may comprise either a single strand or multiple strands of a suitable polymer or metal filament. For example, cord 110 may comprise a braided cable made from 316LVM-type stainless steel wire, with an overall diameter of about 0.5 mm.

The holder 245 also retains and compresses the spring 250. A threaded housing 230 controls the position of holder 245 and thus adjusts the degree of compression and the baseline force on spring 250. The threaded housing 230 has an outer thread, which travels along an inner thread in the anchor 200.

The spring 250 controls the tension in the cord 110. The spring typically comprises a flexible biocompatible material, such as stainless steelor cobalt chrome alloy. Alternatively, device 100 may comprise other sorts of mechanically-loaded elements that create mechanical resistance while deforming, such as a flexible polymer or viscoelastic material, a magnetic element applying mechanical force during movement, or a pneumatic or hydraulic element configured to resist geometrical movement.

The compression of the spring 250 can be adjusted by rotation of the threaded housing230 (as described in greater detail herein below). Turning the threaded housing230 clockwise (assuming the threaded housing to be right-handed) compresses the spring, creating a corresponding "zero-state tension" in cord 110. For example, the zero-state tension may be set to a value in the range between approximately 10 and 15 Newtons, although higher and lower values of tension may also be used, depending on clinical conditions.

The longitudinal position of holder 245 within anchor 200 is controlled by an adjustment mechanism comprising screw 44, which travels along internal thread 46 inside the anchor. For example, turning screw 44 counterclockwise (assuming thread 46 to be right-handed) causes holder 40 to shift longitudinally in the proximal direction, thus drawing cord 30 into anchor 22. In this manner, the length of the cord extending between the anchors is reduced, and the inter-metatarsal angle is reduced accordingly. Alternatively, screw 44 may be turned clockwise to play out the cord and thus reduce the force exerted between the metatarsal bones.

The implantable device is, for example, configured for an intramedullary insertion and provided without said male thread and a nut threadable onto said male thread is also in the scope of the present invention.

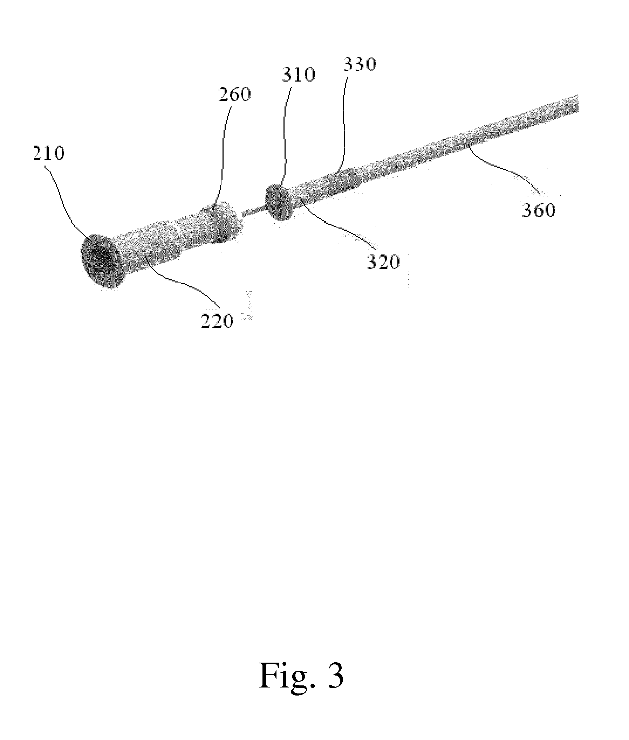

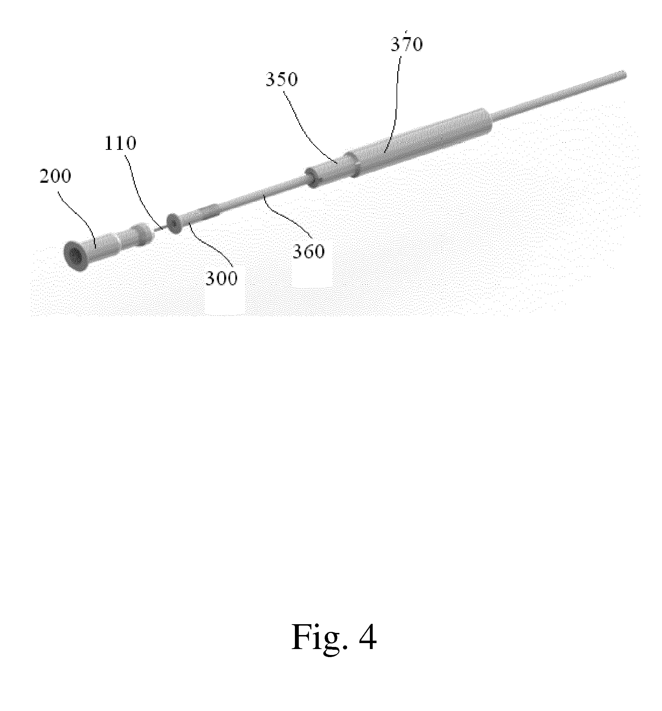

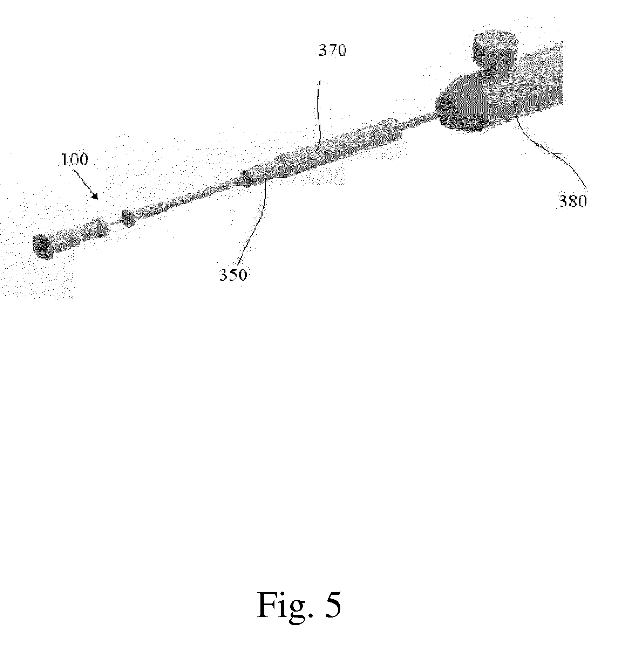

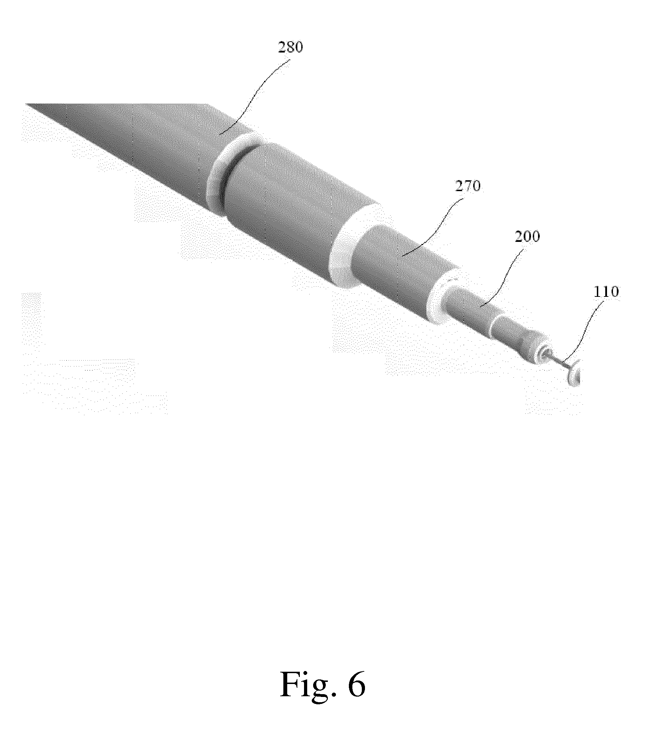

Reference is now made to Figs. 3-7, presenting specific technologic tools. Fig. 3 shows an extension shaft 360 which is screwable into a cylindrical portion 320 of the distal anchor. The extension shaft 360 is used as guide for mounting the nut 350 onto the cylindrical portion 320. Specifically, Fig. 4 illustrates sliding the nut 350 and a locking tool 370 along the extension shaft 360. In Fig. 5, a handle 380 is used for threading the nut 350 for its fixation at the distal anchor. The nut 350 secures the device 100 at the distal side. After threading the nut 350, locking tool 370 held by the handle 380 are removed. Fig. 6 schematically presents a step of adjusting a cord tension force. Practically, a tool configured for rotating the housing 230 (not shown) held by a handle 280 longitudinally displaces the housing 230 within the anchor 200 (not shown).

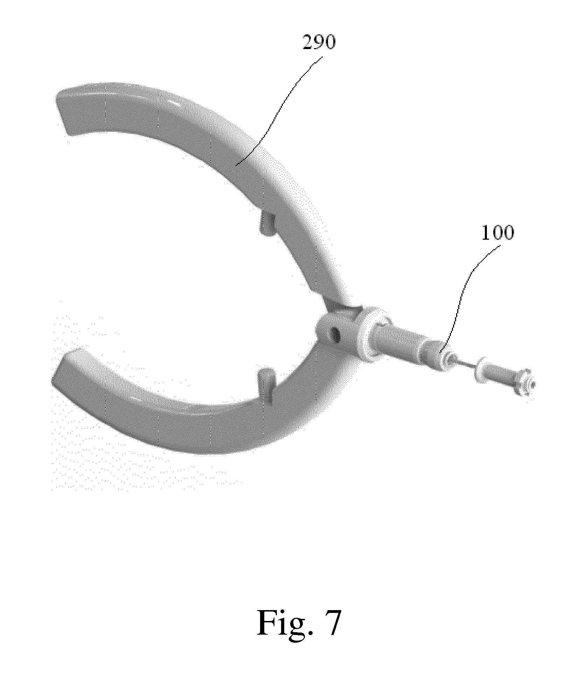

Fig. 7 shows a tool 290 designed for deploying at least one tab into a cortical area of a bone after implanting said anchor into.

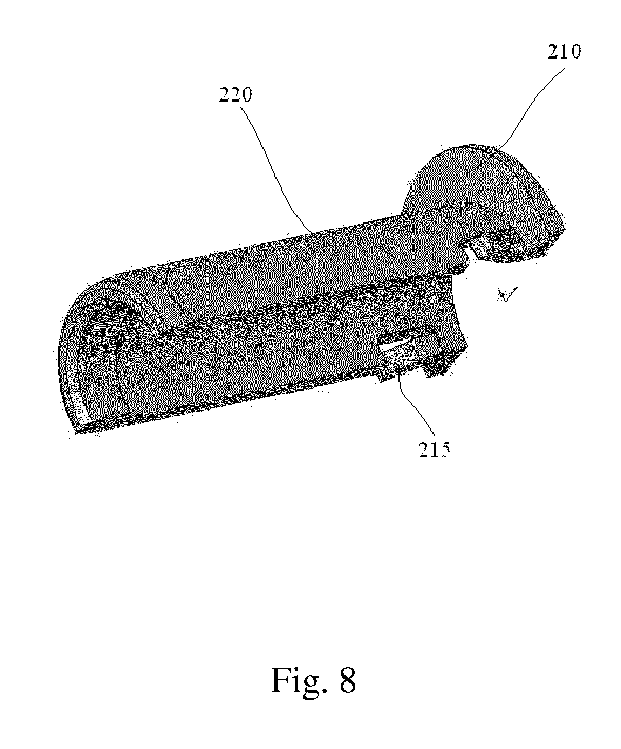

Reference is now made to Fig. 8, schematically presenting a phantom view of a housing of a proximal anchor. A tab 215 is bent (deployed) into the cortical area of a bone (not shown) after implanting said anchor.

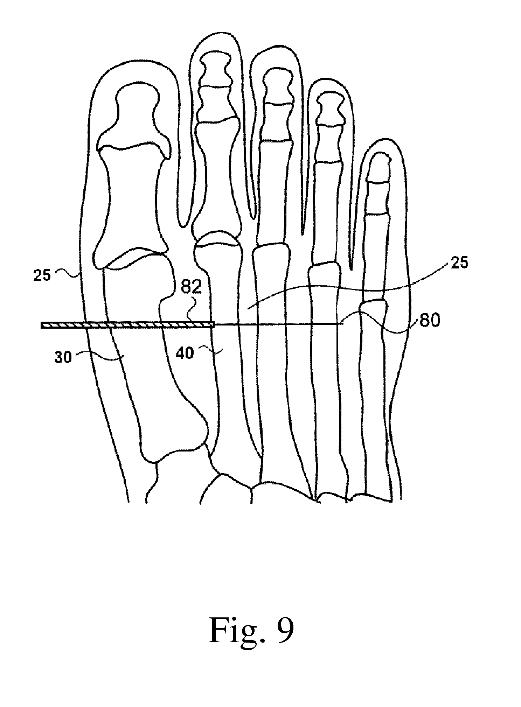

Fig. 9 is a schematic top view of a foot showing a preparatory stage in a procedure for of implanting device 100, in accordance with an embodiment of the present invention. As an initial step, the surgeon may make incisions at locations 25, i.e., at the medial end of the first metatarsal and the lateral end of the second metatarsal. The surgeon may then remove the medial eminence (excess tissue) from the bunion on first metatarsal bone 30 and may cut the adductor tendon away from the first metatarsal bone so that the first and second metatarsal bones can be brought closer together. These surgical steps are known in the art and are outside the scope of the present patent application.

The surgeon first reduces the distance between first and second metatarsal bones 30, 40 by applying force on the first metatarsal toward the second metatarsal, either by hand or using a suitable tool, such as a special-purpose clamp (not shown). Following this step, the surgeon percutaneously drills a K-wire 80 through first and second metatarsal bones 30 and40 using a drill guide (not shown). The K-Wire diameter may be, for example, 1.1 mm. The surgeon then drills through bones 30 and 40 using a cannulated drill 82 over K-wire 80. In the present example, drill 82 has a diameter of 2.8 mm, slightly smaller than the outer diameter of anchor 300.

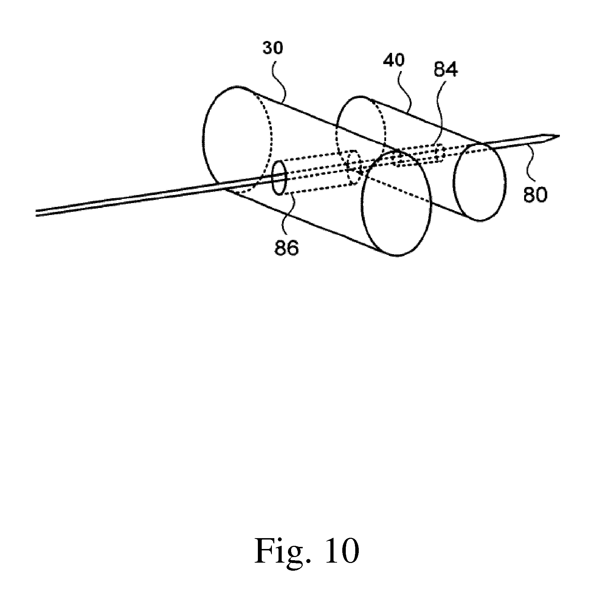

Figs. 10-13 are schematic, pictorial representations of bones 30 and 40, showing successive stages in the surgical procedure, in accordance with an embodiment of the present invention. In Fig. 8, a 2.8 mm bore 84 has been drilled in second metatarsal bone 28. The surgeon then uses a larger drill, for example, 6 mm in diameter, to create a larger bore 86 in first metatarsal bone 30. K-wire 80 remains in the bores at this stage.

To aid in insertion of anchors 200 and 300, a tube 360, such as a plastic pipe or cannulated metal tube (made from 316-type stainless steel, for example), is used as an surgical aid. Tube 360 may have a handle 380 attached to facilitate manipulation by the surgeon.

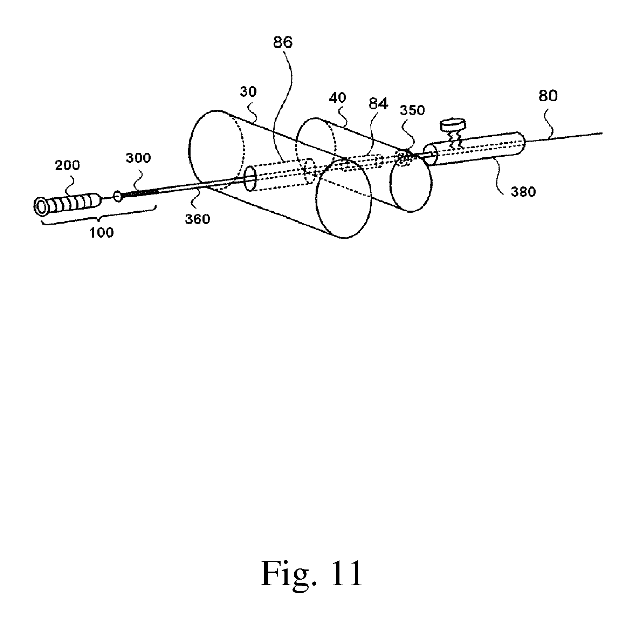

As shown in Fig. 11, the surgeon slides tube 360 over K-wire 80 through bores 84 and 86.Then, the distal end of tube 360is attached to anchor 300. Next, the surgeon pulls the K-wire out of the bores in the distal direction. The surgeon then pulls the tube in the distal direction to draw anchors 300 and 200 through bore 86 in turn, until the anchors are inserted fully into bores 84 and 86, respectively.

In an alternative embodiment, tube 360 may be pre-attached to implant 300 at the factory by laser welding, for example. In this case, handle 380 may be pulled over the distal end tube 360 from bore 86 to 84 and secured to the tube by a screw knob 380.

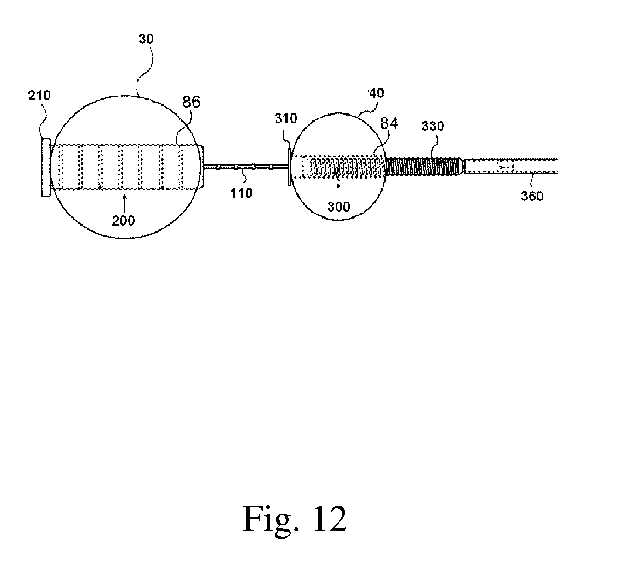

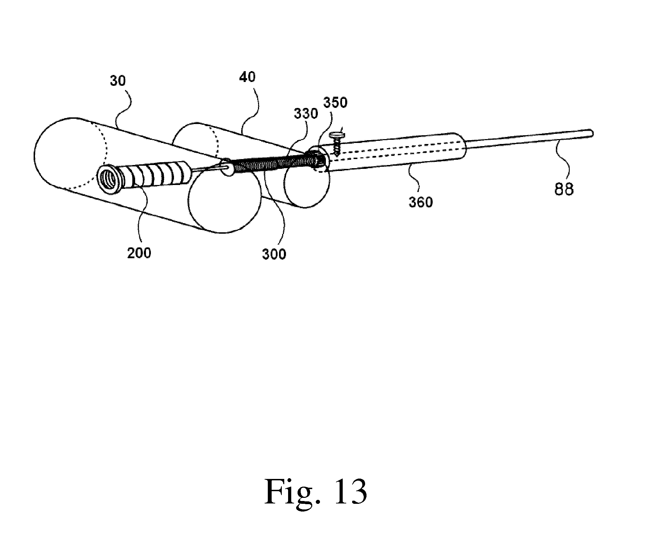

The final positions of the anchors 200 and 300 in bores 86 and 84, respectively, are shown in Fig. 12. Collars 210 and 310 engage the respective surfaces of bones 30 and 40 on the proximal side, while thread 330 and tube 360 protrude outward from bore 84 on the distal side. To secure anchor 300 in place, the surgeon loosens tool 89 from tube 360 and slides nut 350, which resides on tool 89, over tube 350, as shown in Fig. 13. The surgeon then tightens the nut over thread 330 using tool 89 to engage the distal side of bone 40.

Final adjustment of the cord tension force is performed by the special tool 270 (Fig. 6) which is configured for rotating the housing 230 (see Fig. 1) threadly positioned within the proximal anchor 200. The aforesaid anchor 200 is additionally fixated within the first metatarsial by deploying at least one tab into a cortical area of the bone (see Fig. 8).

The cable might be coated with tissue growth promoters such as collagen or growth supporting polymers or a specially roughened surface to promote tissue attachment or alternatively a smooth surface preventing tissue attachment as needed per specific indication.

The tension of the cable acts as a tissue guiding element allowing regeneration of tissue along the cable and establishing a force-sharing function of the cable.

## Claims

1. An implantable device, comprising:
(a) a proximal anchor (200), configured to be implanted inside a first metatarsal; said proximal anchor (200) having a collar (210) larger than the opening in said metatarsal in which said proximal anchor (200) is implanted; said collar (210) preventing cable to bone contact
(b) a distal anchor (300), configured to be implanted inside a second metatarsal, adjacent to said first metatarsal; said distal anchor having a male thread (330);
(c) a nut (350) threadable onto said male thread (330) of the distal anchor (300);
(d) a cord (110) mechanically interconnecting said proximal and distal anchors (200, 300);
(e) a shock absorber comprising an elongate housing (230) threadedly positioned within said proximal anchor (200); wherein said shock absorber accommodates a cord holder (245) mechanically connected to said housing (230) via a damping member (250);
**characterized in that** said cord holder (245) is freely rotatable within said housing (230) to prevent said cord (110) from twist kinking when said housing (230) is rotated to adjust a tension force.

2. The implantable device according to claim 1,wherein said damping member is selected from the group consisting of a spring, a hydraulic piston, a deformable polymer, a deformation energy dampening element, a spring-controlled bellows, spring-controlled telescopic member and any combination thereof.

3. The implantable device according to claim 2, wherein said spring is configured for operation under compression or extension.

4. The implantable device according to claim 1, wherein at least one anchor is provided with at least one tab deployable into a cortical area of a bone after implanting said anchor into.

5. The implantable device according to claim 1, wherein a cord tension force is adjustable due to thread displacement of said shock absorber along said anchor.

6. The implantable device according to claim 1, wherein the male thread is configured for threading the nut at said thread to the rearward of said second metatarsal.

7. The implantable device according to claim 5, wherein said distal anchor has a female thread configured for threading an extension rod for guiding said nut to be treaded onto said distal anchor.

8. The implantable device according to claim 1, wherein said shock absorber comprises a deformable polymer bottleneck member.

9. The implantable device according to claim 1, wherein a position of said anchors implanted in an intramedullary location is selected form the group consisting of longitudinal, oblique and traverse relative to the bone.

10. The implantable device according to claim 1, further comprising
(a) a K-wire drilling guide;
(b) a drilling device.

## Patentansprüche

1. Eine implantierbare Vorrichtung, bestehend aus:
(a) einem proximalen Anker (200), der so gestaltet ist, dass er in einen ersten Mittelfußknochen implantiert werden kann; der besagte proximale Anker (200) verfügt dabei über einen Kranz (210), der größer ist als die Öffnung in besagtem Mittelfußknochen, in den der proximale Anker (200) implantiert wird; der Kranz (210) verhindert einen Kabel-Knochen-Kontakt
(b) einem distalen Anker (300), der so gestaltet ist, dass er in einen zweiten Mittelfußknochen implantiert werden kann, der neben dem ersten Mittelfußknochen liegt; dieser distale Anker verfügt über ein Außengewinde (330);
(c) einer Gewindemutter (350), die auf das Außengewinde (330) des distalen Ankers (300) aufschraubbar ist;
(d) einem Kabel (110), das den proximalen und distalen Anker (200,300) mechanisch verbindet;
(e) einem Stoßdämpfer, bestehend aus einem Längsgehäuse (230), das mit einem Gewinde versehen im proximalen Anker (200) positioniert ist; wobei dieser Stoßdämpfer einen Kabelhalter (245) enthält, der mechanisch mit dem Gehäuse (230) über ein Dämpfungs-Teil (250) verbunden ist;
**dadurch gekennzeichnet, dass** dieser Kabelhalter (245) in diesem Gehäuse (230) frei drehbar ist, um zu verhindern, dass das Kabel (110) sich verdreht und verknotet, wenn besagtes Gehäuse (230) gedreht wird, um eine Zugkraft einzustellen.

2. Die implantierbare Vorrichtung gemäß Anspruch 1, wobei das besagte Dämpfungs-Teil ausgewählt ist aus der Gruppe bestehend aus einer Feder, einem hydraulischen Kolben, einem verformbaren Polymer, einem deformationsenergiedämpfenden Element, einem federgesteuerten Ausgleichselement, federgesteuerten Teleskop-Teil und jeder Kombination daraus.

3. Die implantierbare Vorrichtung gemäß Anspruch 2, wobei die besagte Feder so gestaltet ist, dass sie bei Kompression oder Extension funktioniert.

4. Die implantierbare Vorrichtung gemäß Anspruch 1, wobei mindestens ein Anker vorhanden ist, mit mindestens einer Klappe, die in einen kortikalen Bereich eines Knochens nach der Implantation des besagten Ankers einsetzbar ist.

5. Die implantierbare Vorrichtung gemäß Anspruch 1, wobei eine Kabel-Zugkraft einstellbar ist, aufgrund einer Gewindeverschiebung des Stoßdämpfers entlang des besagten Ankers.

6. Die implantierbare Vorrichtung gemäß Anspruch 1, wobei das Außengewinde so gestaltet ist, dass es die Mutter an diesem Gewinde an die Rückseite des besagten zweiten Mittelfußknochens führt.

7. Die implantierbare Vorrichtung gemäß Anspruch 5, wobei der distale Anker ein Innengewinde hat, das so gestaltet ist, dass es eine Verlängerungsstange aufnimmt, die die Gewindemutter führt, die auf den distalen Anker aufgedreht werden soll.

8. Die implantierbare Vorrichtung gemäß Anspruch 1, wobei der Stoßdämpfer ein verformbares Polymer-Flaschenhals-Teil umfasst.

9. Die implantierbare Vorrichtung gemäß Anspruch 1, wobei eine Position der besagten Anker, die an einer intramedullären Stelle implantiert sind, ausgewählt ist aus der Gruppe bestehend aus längs, schräg und quer zum Knochen.

10. Die implantierbare Vorrichtung gemäß Anspruch 1, darüberhinaus bestehend aus
(a) einer K-Draht-Bohrbüchse;
(b) einer Bohrvorrichtung.

## Revendications

1. Un dispositif implantable, comprenant :
(a) une ancre proximale (200), configurée de façon à être implantée à l'intérieur d'un premier métatarsien, ladite ancre proximale (200) possédant un collier (210) plus grand que ladite ouverture dans ledit métatarsien dans lequel ladite ancre proximale (200) est implantée, ledit collier (210) empêchant un contact câble à os,
(b) une ancre distale (300), configurée de façon à être implantée à l'intérieur d'un deuxième métatarsien, adjacent audit premier métatarsien, ladite ancre distale possédant un filetage mâle (330),
(c) un écrou (350) pouvant être vissé sur ledit filetage mâle (330) de l'ancre distale (300),
(d) un cordon (110) interconnectant mécaniquement lesdites ancres proximale et distale (200, 300),
(e) un amortisseur de chocs comprenant un logement allongé (230) positionné par filetage à l'intérieur de ladite ancre proximale (200), où ledit amortisseur de chocs comprend un porte-cordon (245) mécaniquement raccordé audit logement (230) par l'intermédiaire d'un élément d'amortissement (250),
**caractérisé en ce que** ledit porte-cordon (245) est librement pivotable à l'intérieur dudit logement (230) de façon à empêcher ledit cordon (110) de vriller lorsque ledit logement (230) est pivoté de façon à ajuster une force de tension.

2. Le dispositif implantable selon la Revendication 1, où ledit élément d'amortissement est sélectionné dans le groupe se composant d'un ressort, d'un piston hydraulique, d'un polymère déformable, d'un élément d'amortissement d'énergie de déformation, d'un soufflet commandé par ressort, d'un élément télescopique commandé par ressort, et de toute combinaison de ceux-ci.

3. Le dispositif implantable selon la Revendication 2, où ledit ressort est configuré pour un fonctionnement en compression ou extension.

4. Le dispositif implantable selon la Revendication 1, où au moins une ancre est équipée d'au moins une languette déployable dans une zone corticale d'un os après l'implantation de ladite ancre.

5. Le dispositif implantable selon la Revendication 1, où une force de tension de cordon est ajustable du fait d'un déplacement de filetage dudit amortisseur de chocs le long de ladite ancre.

6. Le dispositif implantable selon la Revendication 1, où le filetage mâle est configuré de façon à visser l'écrou au niveau dudit filetage vers l'arrière dudit deuxième métatarsien.

7. Le dispositif implantable selon la Revendication 5, où ladite ancre distale possède un filetage femelle configuré de façon à visser une tige d'extension destinée au guidage dudit écrou à visser sur ladite ancre distale.

8. Le dispositif implantable selon la Revendication 1, où ledit amortisseur de chocs comprend un élément de goulot d'étranglement polymère déformable.

9. Le dispositif implantable selon la Revendication 1, où une position desdites ancres implantées dans un emplacement intramédullaire est sélectionnée dans le groupe se composant de direction longitudinale, oblique et transverse par rapport à l'os.

10. Le dispositif implantable selon la Revendication 1, comprenant en outre
(a) un guide de perçage à broche de Kirschner,
(b) un dispositif de perçage.
